# EUROPEAN PATENT SPECIFICATION

(11) **EP 3 476 369 B1**
(45) Date of publication and mention of the grant of the patent: **12.03.2025**
(21) Application number: 17819130.0
(22) Date of filing: 19.06.2017
(51) Int. Cl.: A61F 2/07

(54) **MEMBRANE-COATED STENT AND MANUFACTURING METHOD THEREOF**
MEMBRANBESCHICHTETER STENT UND HERSTELLUNGSVERFAHREN DAFÜR
STENT INTRAVASCULAIRE ET SON PROCÉDÉ DE FABRICATION.

(30) Priority: 28.06.2016 CN 201610489191
(43) Date of publication of application: 01.05.2019
(73) Proprietor: Shanghai VasoLutions MedTech Co., Ltd., Shanghai 201318 (CN)
(72) Inventor: XIA, Shun, Shanghai 201318 (CN); XING, Zhikai, Shanghai 201318 (CN); LIU, Mengqin, Shanghai 201318 (CN); YUAN, Zhenyu, Shanghai 201318 (CN); ZHU, Qing, Shanghai 201318 (CN); LI, Zhonghua, Shanghai 201318 (CN)
(74) Representative: Patentship Patentanwaltsgesellschaft mbH
(86) International application number: PCT/CN2017/089021
(87) International publication number: WO 2018/001133

(56) References cited:
- WO-A1-2006/062957
- WO-A1-2017/184381
- CN-A- 101 176 686
- CN-A- 101 627 933
- CN-A- 103 720 529
- US-A1- 2009 099 642
- US-A1- 2010 082 093
- US-A1- 2014 142 683

## Description

### Technical Field

The present invention relates to a stent-graft and methods of fabricating it.

### Background

In recent years, stent-grafts using expanded polytetrafluoroethylene (ePTFE)-based graft materials have been widely recognized in the field of implantable medical devices, thanks to their advantages including excellent biocompatibility and extra slipperiness. Indications for stent-grafts of this type include peripheral vascular embolization, aorta and branch dissections, true aneurysms in the aorta and branches, false aneurysms in the aorta and branches as well as penetrating ulcers in the aorta and branches. This technique greatly reduces operative mortality and postoperative complications, creates less surgical trauma and allows faster patient recovery. Its therapeutic mechanism is to use a special delivery system to deliver a stent-graft to the target lesion site and then cause it to expand to open a blood vessel blocked by a clot or isolate an aneurysm from the bloodstream, eliminating the risk of death due to massive bleeding caused by an aneurysmal rupture or of aneurysmal compression of surrounding tissues or organs. At present, there have been established products in the world, such as the foreign brands Gore Viabahn and Bard Fluency and the domestic brands Aegis, Ankura, etc.

A conventional stent-graft is often crimped and pushed/pulled into a delivery system and deployed from the delivery system by retracting the stent-graft backward or advancing the stent-graft forward. However, since ePTFE-based materials tend to creep under stress, the conventional loading method is less suitable for stent-grafts using such materials. Specifically, such a stent-graft will deform and remarkably shrink compared to its nominal dimensions upon exposure to a considerable stress occurring during the conventional approach.

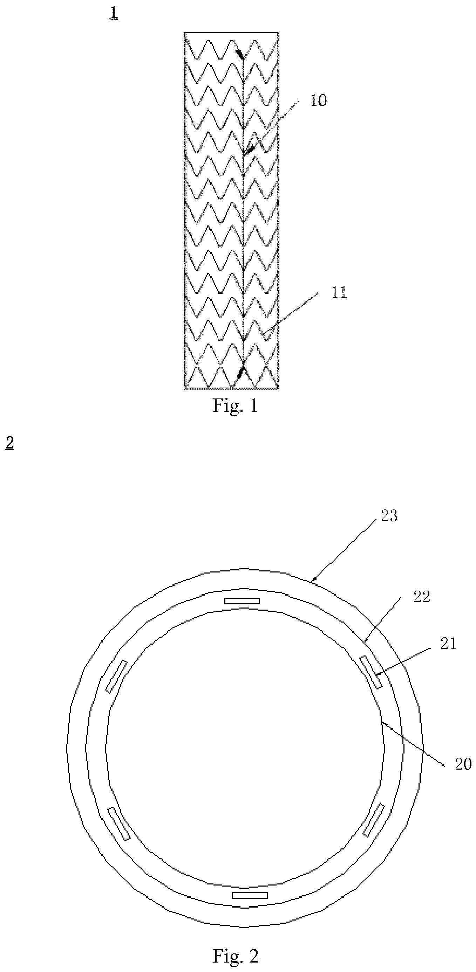

In order to overcome this problem, most stent-graft products available on the contemporary market are structurally modified to achieve higher loading performance at the price of compromised flexibility. For example, some of them adopt reinforcing ribs usually in the form of nickel-titanium alloy filaments connecting both ends of the stent. Fig. 1 shows such a stent-graft 1 including a reinforcing rib 10. The reinforcing rib 10 has tiny stainless steel eyelets through which it is connected to a stent 11. This structure has enhanced axial rigidity which can prevent the stent-graft 1 from experiencing permanent length contraction or expansion. However, the reinforcing rib makes the stent-graft less flexible and thus significantly narrows its application (e.g., fabricating it inapplicable to tortuous blood vessels). In addition, it increases the bulk of the stent-graft, which is unfavorable to the loading.

US 2009/099642 A1 discloses a stent graft which includes a stent and a graft engaged with the stent. The graft can include an inner surface and an outer surface. Further, at least one of the inner surface and the outer surface can include a plurality of protrusions as viewed in cross section extending through a longitudinal axis.

Therefore, there is an urgent need in the art for a stent-graft having both good flexibility and high strength.

### Summary of the Invention

It is an objective of the present invention to overcome the problems with the conventional stent-grafts by presenting a stent-graft with both good flexibility and high strength and methods of fabricating such a stent-graft, as set forth in the appended claims.

To this end, the provided stent-graft includes a first graft and a second graft surrounding the first graft, wherein a filament and a stent are disposed between the first and second grafts, and wherein the filament and the first and second grafts are all fastened to the stent.

Optionally, in the stent-graft, the filament may be formed of ePTFE, PTFE or FEP.

Optionally, in the stent-graft, a thickness of the filament measured radially with respect to the stent-graft may be smaller than a width of the filament measured circumferentially with respect to the stent-graft.

The stent-graft has a plurality of filaments, the plurality of filaments spiral in opposite directions at a same angle with respect to a central axis of the stent-graft.

Optionally, in the stent-graft, the filament may be arranged between the first graft and the stent, interlaced within the stent and/or disposed between the stent and the second graft.

Optionally, in the stent-graft, the first and second grafts may be both formed of ePTFE.

The present invention also provides a method of fabricating a stent-graft, including:
winding a first graft over a bobbin;
arranging a filament and a stent outside the first graft;
winding a second graft over the filament and the stent, thereby forming the stent-graft; and
subjecting the stent-graft to a shaping process and removing the shaped stent-graft from the bobbin.

Optionally, in the method, the filament may be formed of ePTFE, PTFE or FEP.

Optionally, in the method, a thickness of the filament measured radially with respect to the stent-graft may be smaller than a width of the filament measured circumferentially with respect to the stent-graft.

In the method, there are a plurality of filaments, the plurality of filaments spiral in opposite directions at a same angle with respect to a central axis of the stent-graft.

Optionally, in the method, arranging the filament and the stent outside the first graft may include:
arranging the filament outside the first graft; and
sleeving the stent over the filament;
   or
sleeving the stent over the first graft; and
interlacing the filament within the stent;
   or
sleeving the stent over the first graft; and
arranging the filament outside the stent.

Optionally, before subjecting the stent-graft to a shaping process, the method may further include:
wrapping the stent-graft with a dust-proof film; and
sleeving a crimp tube over the dust-proof film.

Optionally, in the method, the first and second grafts may be both formed of ePTFE.

The present invention also provides another method of fabricating a stent-graft, including:
fastening a filament to a stent; and
forming a first graft and a second graft inside and outside both the filament and the stent, respectively.

Optionally, in the method, the first graft and the second graft may be respectively formed inside and outside both the filament and the stent by an electrospinning process.

According to the present invention, the filament added between the two grafts is flexible and strong and thus imparts both good flexibility and high strength to the stent-graft. As a result, creep deformation of the stent-graft is effectively avoided at
the price of a minor increase in its outer diameter, while facilitating the loading of the stent-graft into a delivery system. The facilitation includes considerably helping in keeping the intactness of the stent-graft during the loading and release processes and providing a possibility of loading it in smaller catheters so as to leave more room for surgical operations of the physician.

### Brief Description of The Drawings

Fig. 1 is a structural schematic of a conventional stent-graft.
Fig. 2 is a schematic cross-sectional view of a stent-graft according to Example 1 of the present disclosure.
Fig. 3 is a schematic front view of the stent-graft according to said Example 1.
Fig. 4 is a schematic illustration of the stent-graft according to said Example 1 in use.
Fig. 5 is another schematic illustration of the stent-graft according to said Example 1 in use.
Fig. 6 is a flowchart of a method for fabricating the stent-graft according to the present invention.
Fig. 7 is a schematic front view of a stent-graft according to Example 3 of the present disclosure.
Fig. 8 is a schematic front view of a stent-graft according to the present invention.

### Detailed Description of Preferred Embodiments

A few embodiment examples of the present invention will be described in detail with reference to the accompanying drawings. Features and advantages of the invention will be more apparent from the following detailed description, and from the appended claims. Note that these figures are presented in a very simple form not necessarily drawn to scale, with the only intention of facilitating convenience and clarity in explaining the examples of the invention. In particular, the figures generally give emphasis on different details and are accordingly drawn to different scales.

### Example 1

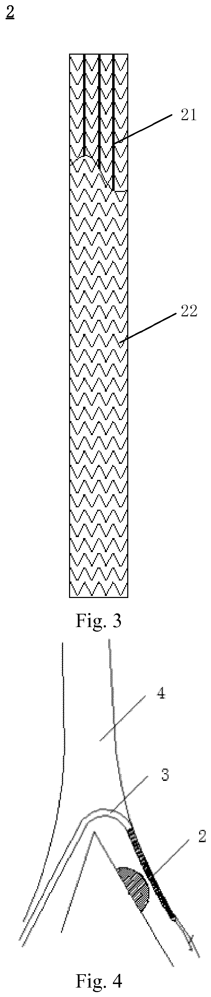

Reference is now made to Figs. 2 and 3, wherein Fig. 2 is a schematic cross-sectional view of a stent-graft according to Example 1, and Fig. 3 is a schematic front view of a stent-graft according to Example 1. As shown in Figs. 2 and 3, the stent-graft 2 according to Example 1 includes a first graft 20 and a second graft 23 surrounding the first graft 20. Disposed between the first and second grafts 20, 23 are filaments 21 and a stent 22. The filaments 21, the first and second grafts 20, 23 are all fastened to the stent 22. In this example, both the first and second grafts 20, 23 are ePTFE grafts, and the stent 22 is a metal stent.

With continued reference to Figs. 2 and 3, in this example, the filaments 21 are
disposed between the first graft 20 and the stent 22. That is, the filaments 21 are arranged within (inside) the stent.

Preferably, the filaments 21 are ePTFE, PTFE or FEP filaments. Additionally, the filaments 21 are flat strips, i.e., each of the filaments 21 has a thickness, measured radially with respect to the stent-graft 2, that is smaller than a width of the corresponding filament 21 measured circumferentially with respect to the stent-graft 2.

The filaments 21 are disposed between the two grafts (i.e., the first and second grafts 20, 23). The good flexibility and high strength of the filaments 21 impart both good flexibility and high strength to the stent-graft 2. Further, as the filaments 21 are flat strips, the stent-graft 2 can have a small outer diameter, so that the stent-graft 2 is easy to use.

With continued reference to Figs. 2 and 3, in this Embodiment, the number of the filaments 21 is more than one, and each of the filaments 21 extends parallel to a central axis of the stent-graft 2. Additionally, the multiple filaments 21 are evenly distributed circumferentially with respect to the stent-graft 2. As a result, good flexibility and high strength are obtained throughout the whole stent-graft 2.

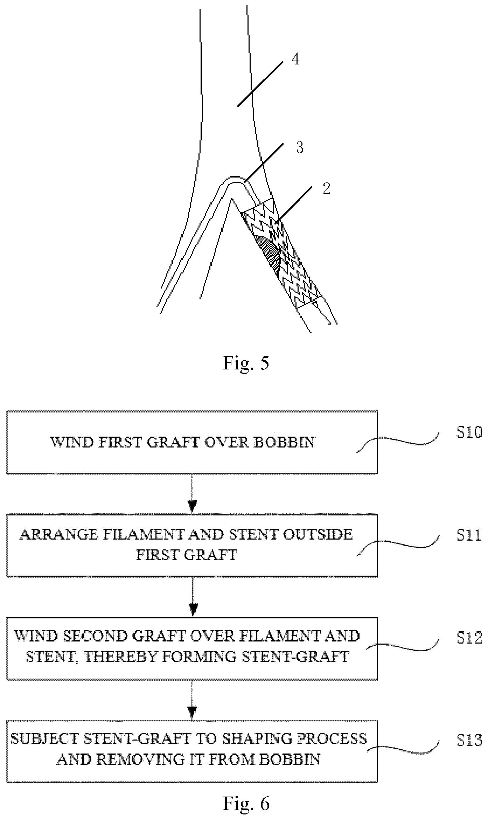

Reference is now made to Figs. 4 and 5, schematic illustrations of the stent-graft in use according to Example 1. As shown in Figs. 4 and 5, during use, the stent-graft 2 is loaded into a delivery catheter 3, advanced by the delivery catheter 3 to a target site, such as an occluded portion of the iliac artery 4, and then the stent-graft 2 is deployed.

In Example
1, there is also provided a corresponding method for fabricating the stent-graft 2. Fig. 6 is a flowchart of the method for fabricating the stent-graft according to Embodiment 1 of the present invention. As shown in Fig. 6, specifically, the stent-graft 2 may be fabricated in the following steps.

In step S10, a first graft is wound over a bobbin. The first graft is an ePTFE film and may be wound one or more turns as practically desired. Moreover, a thickness of the first graft may be adjustable as practically desired.

In this Embodiment, the size of the bobbin can be selected according to a design inner diameter of the stent-graft being fabricated. For example, for a stent-graft with an inner diameter of 16 mm, a cylindrical bobbin having a diameter of 16 mm may be chosen. Preferably, before the first graft is wounded over the bobbin, the bobbin can be washed with alcohol in order to ensure its cleanness. The alcohol may be selected as a 75% alcohol solution. After the washing of the bobbin, the bobbin may be placed in the air until it is dried. More preferably, after the bobbin is washed with alcohol and before the first graft is wound thereon, the bobbin may be wrapped with a dust-proof film, in order to ensure its even higher cleanness, which can lead to higher quality of the stent-graft. The dust-proof film may be a piece of aluminum, tin or silver foil-backed paper.

In step S11, the filaments and the stent are arranged outside the first graft. In this Embodiment, the filaments are arranged inside the stent (i.e., between the first graft and the stent). Accordingly, the arrangement of the filaments and the stent outside the first graft may include the steps of: arranging the filaments outside the first graft; and sleeving the stent over the filaments. This will be described in further detail below.

At first, the filaments are attached to the outer surface of the first graft. Preferably, the filaments are made of ePTFE, PTFE or FEP. The filaments may be arranged into stacks each containing one or more of them to achieve a total thickness as practically desired. In general terms, for an application requiring high strength/tensile and creep resistance of the stent-graft, the multiple filaments may be arranged into a high density of stacks each containing one or more of them to achieve a large total thickness, and for an application not demanding high strength/tensile and creep resistance but requiring high flexibility of the stent-graft, fewer filaments may be used without being stacked so that the density and total thickness of them can be reduced.

Preferably, the filaments are flat strips so that the stent-graft has a smaller outer diameter and is less bulky.

In Example
1, the number of the filaments is greater than one, and the filaments are circumferentially distributed evenly with respect to the bobbin, i.e., evenly along a circumference of the bobbin (more precisely, an outer circumference of the first graft). Moreover, the filaments extend axially with respect to the bobbin, i.e., having their central axes all parallel to a central axis of the bobbin. For example, six filaments having a length of 15 cm (i.e., the stent-graft being fabricated has a length of 15 cm), a width of 1 mm (i.e., the filaments are 1 mm wide along the circumferential direction of the bobbin) and a thickness of 0.02 mm (i.e., the filaments are 0.02 mm thick along the radial direction of the bobbin) are circumferentially arranged evenly with respect to the bobbin so that every adjacent two of them are spaced apart from each other by an angular pitch of 60 degrees.

Next, the stent is sleeved over the filaments. The stent may be made of a stainless steel, a cobalt-chromium alloy, a nickel-titanium alloy or another material with desired biocompatibility and mechanical properties. Specifically, the stent sleeved over the filaments may be a mesh frame having a wave pattern and made of a cobalt chromium alloy, a nickel-titanium alloy or the like. Generally, such mesh frames are made beforehand and stored, and upon the fabrication of the stent-graft, a suitable one of them is selected and directly used. In step S12, a second graft is wound around the filaments and the stent to complete the stent-graft. The second graft may be wound one or more turns to achieve a thickness of the second graft as practically desired.

At least, in step S13, the stent-graft is subjected to a shaping process and then removed from the bobbin. In this Embodiment, the shaping process performed on the stent-graft is accomplished with a heat treatment. In other embodiments consistent with the present application, it may also be accomplished with a high-pressure heat treatment, a vacuum extrusion process or an adhesive. More preferably, prior to the performance of the shaping process on the stent-graft, the stent-graft may be wrapped with a dust-proof film and then with a crimp tube. Specifically, the dust-proof film may be a piece of aluminum, tin or silver foil-backed paper, while the crimp tube may be selected as a PTFE heat-shrink tube or the like. The heat treatment may be performed on the stent-graft in an oven at a temperature of 350°C to 400°C for 10 minutes to 15 minutes. In this Embodiment, wrapping the stent-graft with the dust-proof film can prevent the stent-graft from being contaminated with dust or the like, thereby ensuring its quality and reliability. The crimp tube sleeved over the dust-proof film allows the stent-graft to be uniformly pressurized during the shaping (curing) process, thus further increasing the quality and reliability of the stent-graft.

The stent-graft resulting from the above process has both good flexibility and high strength, and can be used as desired after it is removed from the bobbin.

### Example 2

Referring back to Figs. 2 and 3, Example 2 differs from Example 1 in that,
in Example
1, the filaments 21 are arranged between the first graft 20 and the stent 22, while in Example
2, the filaments 21 are interlaced within the stent 22. In this case,
the filaments 21 are often partially inside the stent 22 and partially outside the stent 22, i.e., the filaments 21 are interlaced within the stent 22. Accordingly, the filaments of Embodiment 2 are disposed in a different manner from those of Example 1.

In Example
2, arranging the filaments and the stent outside the first graft particularly includes the two steps of: sleeving the stent over the first graft; and interlacing the filaments within the stent. The stent may include multiple wave-shaped annular wires (mesh frame) arranged coaxially side-by-side, and each of the filaments may be interlaced over the first annular wire, under the second, over the third, and so forth. That is, each of them extends alternately inside and outside the stent (i.e., going alternately up and down the annular wires).

The resulting stent-graft also has both good flexibility and high strength.

In other examples,
the filaments 21 may also be all disposed outside the stent 22, i.e., the filaments 21 are disposed between the stent 22 and the second graft 23. In this case, arranging the filaments and the stent outside the first graft particularly includes the two steps of: sleeving the stent over the first graft; and attaching the filaments to the outside of the stent.

### Example 3

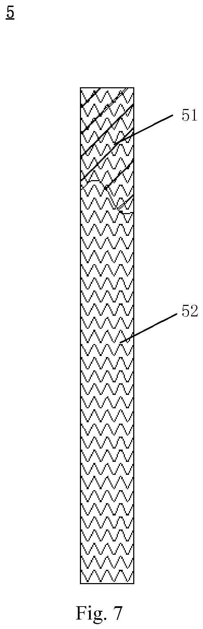

Reference is now made to Fig. 7, a schematic front view of a stent-graft according to Example 3.
As shown in Fig. 7, the stent-graft 5 includes a first graft (not shown in Fig. 7) and a second graft (not shown in Fig. 7) surrounding the first graft. Filaments 51 and a stent 52 are disposed between the first and second grafts, and each of the filaments 51 terminates at both ends of the stent 52. In this example, the first and second grafts are both ePTFE grafts, and the stent 52 is made of a cobalt chromium alloy.

Example 3 differs from Example 1 in that, in Embodiment 1, the filaments extend parallel to the central axis of the bobbin and are circumferentially distributed evenly with respect to the bobbin, while in Example 3, the filaments spiral outside the first graft axially with respect to the bobbin. Accordingly, the filaments of Example 3 are disposed in a different manner from those of Example 1.

In particular, in Example
3, arranging the filaments outside the first graft include the steps of: spirally winding the filaments outside the first graft in the same direction at the same angle with respect to the central axis of the bobbin. Preferably, each of the filaments spirals at an angle of 45°with respect to the central axis of the bobbin.

In other examples, only one such filament may be spirally wound outside the first graft. Similarly, this filament may spiral at an angle of 45°with respect to the central axis of the bobbin.

In generally, for an application requiring high strength/tensile and creep resistance of the stent-graft, multiple said filaments may be used. For an application not demanding high strength/tensile and creep resistance but requiring high flexibility of the stent-graft, only one such filament may be used.

Present invention

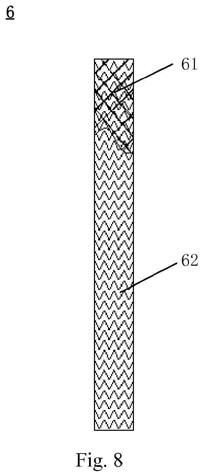

Reference is now made to Fig. 8, a schematic front view of a stent-graft according to the present invention. As shown in Fig. 8, the stent-graft 6 includes a first graft (not shown in Fig. 8) and a second graft (not shown in Fig. 8) surrounding the first graft. Filaments 61 and a stent 62 are disposed between the first and second grafts, and each of the filaments 61 terminates at both ends of the stent 62. In this Embodiment, the first and second grafts are both ePTFE grafts, and the stent 62 is made of a nickel-titanium alloy.

The present invention differs from Example 3 in that, in Example 3, the filaments spiral outside the first graft in the same direction at the same angle with respect to the central axis of the bobbin, while in the present invention,
the filaments spiral outside the first graft in opposite directions at the same angle with respect to the central axis of the bobbin.

For example, two said filaments may be spirally wound outside the first graft both in Example 3 and the present invention, wherein the two filaments both lead from the right upper corner of the bobbin in Example
3 (i.e., the unidirectional spiraling configuration), while in the present invention,
one filament leads from the right upper corner of the bobbin and the other from the left upper corner thereof (i.e., the bidirectional spiraling configuration).

In other embodiments, the stent-graft may also be fabricated by a method including: fastening the filaments to the stent; and forming the first and second grafts inside and outside both the filaments and the stent, respectively. The formation of the first and second grafts inside and outside both the filament and the stent may be accomplished by an electrospinning process.

In summary, the filament added between the two grafts is flexible and strong and thus imparts both good flexibility and high strength to the stent-graft. As a result, creep deformation of the stent-graft can be effectively avoided at the price of a minor increase in its outer diameter, while facilitating the loading of the stent-graft into a delivery system.

The description presented above is merely that of a few preferred embodiments of the present invention and does not limit the scope thereof in any sense. Any and all changes and modifications made by those of ordinary skill in the art based on the above teachings fall within the scope as defined in the appended claims.

## Claims

1. A stent-graft (6), comprising a first graft (20) and a second graft (23) surrounding the first graft (20), wherein a plurality of filaments (61) and a stent (62) are disposed between the first graft (20) and the second graft (23) in a radial direction, and wherein the plurality of filaments (61), the first graft (20) and the second graft (23) are all fastened to the stent,
**characterized in that**:
the plurality of filaments (61) each spiral axially with respect to the stent-graft (6), the plurality of filaments (61) spiral in opposite directions at a same angle with respect to the central axis of the stent-graft (6), and the plurality of filaments (61) are configured to impart both good flexibility and high strength to the stent-graft.

2. The stent-graft (6) of claim 1, wherein the filament is formed of ePTFE, PTFE or FEP.

3. The stent-graft (6) of claim 1, wherein a thickness of the filament measured radially with respect to the stent-graft (6) is smaller than a width of the filament measured circumferentially with respect to the stent-graft (6).

4. The stent-graft (6) of any one of claims 1 to 3, wherein the plurality of filaments are arranged between the first graft (20) and the stent, interlaced within the stent (62) and/or disposed between the stent (62) and the second graft (23).

5. The stent-graft (6) of any one of claims 1 to 3, wherein the first graft (20) and the second graft (23) are both formed of ePTFE.

6. A method of fabricating a stent-graft (6), comprising:
fastening a plurality of filaments (61) to a stent; and
forming a first graft (20) and a second graft (23) inside and outside both the plurality of filaments (61) and the stent, respectively,
**characterized in that**:
the plurality of filaments (61) each spiral axially with respect to the stent-graft (6), the plurality of filaments (61) spiral in opposite directions at a same angle with respect to the central axis of the stent-graft (6), and the plurality of filaments (61) are configured to impart both good flexibility and high strength to the stent-graft.

7. The method of claim 6, wherein the step of forming a first graft (20) and a second graft (23) comprises:
winding a first graft (20) over a bobbin;
arranging the plurality of filaments and the stent (62) outside the first graft (20); and
winding a second graft (23) over the plurality of filaments and the stent, thereby forming the stent-graft (6),
the method of fabricating a stent-graft (6) further comprising subjecting the stent-graft (6) to a shaping process and removing the shaped stent-graft (6) from the bobbin.

8. The method of claim 7, wherein arranging the plurality of filaments and the stent (62) outside the first graft (20) comprises:
arranging the plurality of filaments outside the first graft (20); and
sleeving the stent (62) over the plurality of filaments.

9. The method of claim 7, wherein arranging the plurality of filaments and the stent (62) outside the first graft (20) comprises:
sleeving the stent over the first graft (20); and
interlacing the plurality of filaments within the stent (62).

10. The method of claim 7, wherein arranging the plurality of filaments and the stent (62) outside the first graft (20) comprises:
sleeving the stent over the first graft (20); and
arranging the plurality of filaments outside the stent (62).

11. The method of claim 7, wherein before subjecting the stent-graft (6) to a shaping process, the method further comprises:
wrapping the stent-graft (6) with a dust-proof film; and
sleeving a crimp tube over the dust-proof film.

12. The method of claim 6, wherein the first graft (20) and the second graft (23) are formed by an electrospinning process.

## Patentansprüche

1. Stent-Transplantat (6), umfassend ein erstes Transplantat (20) und ein zweites Transplantat (23), welches das erste Transplantat (20) umgibt, wobei eine Vielzahl von Filamenten (61) und ein Stent (62) zwischen dem ersten Transplantat (20) und dem zweiten Transplantat (23) in radialer Richtung angeordnet sind, und wobei die Vielzahl von Filamenten (61), das erste Transplantat (20) und das zweite Transplantat (23) alle am Stent befestigt sind, **dadurch gekennzeichnet, dass**:
die Vielzahl von Filamenten (61) jeweils axial spiralförmig in Bezug auf das Stent-Transplantat (6) verlaufen, die Vielzahl von Filamenten (61) spiralförmig in entgegengesetzte Richtungen in einem gleichen Winkel in Bezug auf die Mittelachse des Stent-Transplantats (6) verlaufen, und die Vielzahl von Filamenten (61) ausgebildet sind, dem Stent-Transplantat sowohl gute Flexibilität als auch hohe Festigkeit zu verleihen.

2. Stent-Transplantat (6) nach Anspruch 1, wobei das Filament aus ePTFE, PTFE oder FEP besteht.

3. Stent-Transplantat (6) nach Anspruch 1, wobei eine radial zum Stent-Transplantat (6) gemessene Dicke des Filaments kleiner als eine in Umfangsrichtung zum Stent-Transplantat (6) gemessene Breite des Filaments ist.

4. Stent-Transplantat (6) nach einem der Ansprüche 1 bis 3, wobei die Vielzahl von Filamenten zwischen dem ersten Transplantat (20) und dem Stent angeordnet, innerhalb des Stents (62) verflochten und/oder zwischen dem Stent (62) und dem zweiten Transplantat (23) angeordnet sind.

5. Stent-Transplantat (6) nach einem der Ansprüche 1 bis 3, wobei das erste Transplantat (20) und das zweite Transplantat (23) beide aus ePTFE gebildet sind.

6. Verfahren zur Herstellung eines Stent-Transplantats (6), umfassend:
Befestigen einer Vielzahl von Filamenten (61) an einem Stent; und
Bilden eines ersten Transplantates (20) und eines zweiten Transplantates (23) innerhalb und außerhalb sowohl der Vielzahl von Filamenten (61) als auch des Stents,
**dadurch gekennzeichnet, dass**:
die Vielzahl von Filamenten (61) jeweils axial spiralförmig in Bezug auf das Stent-Transplantat (6) verlaufen, die Vielzahl von Filamenten (61) spiralförmig in entgegengesetzte Richtungen in einem gleichen Winkel in Bezug auf die Mittelachse des Stent-Transplantats (6) verlaufen, und die Vielzahl von Filamenten (61) ausgebildet sind, dem Stent-Transplantat sowohl gute Flexibilität als auch hohe Festigkeit verleihen.

7. Verfahren nach Anspruch 6, wobei der Schritt des Bildens eines ersten Transplantates (20) und eines zweiten Transplantates (23) umfasst:
Wickeln eines ersten Transplantates (20) um eine Spule;
Anordnen der Vielzahl von Filamenten und des Stents (62) außerhalb des ersten Transplantats (20); und
Wickeln eines zweiten Transplantates (23) über die Vielzahl von Filamenten und den Stent, wodurch das Stent-Transplantat (6) gebildet wird,
wobei das Verfahren zur Herstellung eines Stent-Transplantats (6) weiterhin Unterziehen des Stent-Transplantats (6) einem Formungsprozess und Entfernen des geformten Stent-Transplantats (6) von der Spule umfasst.

8. Verfahren nach Anspruch 7, wobei Anordnen der Vielzahl von Filamenten und des Stents (62) außerhalb des ersten Transplantats (20) umfasst:
Anordnen der Vielzahl von Filamenten außerhalb des ersten Transplantates (20); und
Überziehen des Stents (62) über die Vielzahl von Filamenten.

9. Verfahren nach Anspruch 7, wobei das Anordnen der Vielzahl von Filamenten und des Stents (62) außerhalb des ersten Transplantats (20) umfasst:
Überziehen des Stents über das erste Transplantat (20); und
Verflechten der Vielzahl von Filamenten innerhalb des Stents (62).

10. Verfahren nach Anspruch 7, wobei das Anordnen der Vielzahl von Filamenten und des Stents (62) außerhalb des ersten Transplantats (20) umfasst:
Überziehen des Stents über das erste Transplantat (20); und
Anordnen der Vielzahl von Filamenten außerhalb des Stents (62).

11. Verfahren nach Anspruch 7, wobei das Verfahren, vor Unterziehen des Stent-Transplantats (6) einem Formungsprozess, weiterhin umfasst:
Umwickeln des Stent-Transplantats (6) mit einer Staubschutzfolie; und
Überziehen eines Crimprohrs über die Staubschutzfolie.

12. Verfahren nach Anspruch 6, wobei das erste Transplantat (20) und das zweite Transplantat (23) durch einen Elektrospinnprozess gebildet werden.

## Revendications

1. Stent-greffe (6), comprenant un premier greffon (20) et un second greffon (23) entourant le premier greffon (20), dans lequel une pluralité de filaments (61) et un stent (62) sont disposés entre le premier greffon (20) et le second greffon (23) dans une direction radiale, et dans lequel la pluralité de filaments (61), le premier greffon (20) et le second greffon (23) sont tous fixés au stent,
**caractérisé en ce que** :
la pluralité de filaments (61) s'enroulent chacun axialement par rapport au stent-greffe (6), la pluralité de filaments (61) s'enroulent dans des directions opposées sous un même angle par rapport à l'axe central du stent-greffe (6), et la pluralité de filaments (61) sont configurés pour conférer à la fois une bonne flexibilité et une résistance élevée au stent-greffe.

2. Stent-greffe (6) selon la revendication 1, dans lequel le filament est formé d' ePTFE, de PTFE ou de FEP.

3. Stent-greffe (6) selon la revendication 1, dans lequel une épaisseur du filament mesurée radialement par rapport au stent-greffe (6) est inférieure à une largeur du filament mesurée circonférentiellement par rapport au stent-greffe (6).

4. Stent-greffe (6) selon l'une quelconque des revendications 1 à 3, dans lequel la pluralité de filaments sont disposés entre le premier greffon (20) et le stent, entrelacés à l'intérieur du stent (62) et/ou disposés entre le stent (62) et le second greffon (23).

5. Stent-greffe (6) selon l'une quelconque des revendications 1 à 3, dans lequel le premier greffon (20) et le second greffon (23) sont tous deux formés d' ePTFE.

6. Procédé de fabrication d'une endoprothèse (6), comprenant :
fixer une pluralité de filaments (61) à un stent ; et
former une première greffe (20) et une seconde greffe (23) à l'intérieur et à l'extérieur de la pluralité de filaments (61) et du stent, respectivement,
**caractérisé en ce que** :
la pluralité de filaments (61) s'enroulent chacun axialement par rapport au stent-greffe (6), la pluralité de filaments (61) s'enroulent dans des directions opposées sous un même angle par rapport à l'axe central du stent-greffe (6), et la pluralité de filaments (61) sont configurés pour conférer à la fois une bonne flexibilité et une résistance élevée au stent-greffe.

7. Procédé selon la revendication 6, dans lequel l'étape de formation d'un premier greffon (20) et d'un second greffon (23) comprend :
enrouler un premier greffon (20) sur une bobine ;
disposer la pluralité de filaments et le stent (62) à l'extérieur du premier greffon (20) ; et
enrouler une seconde greffe (23) sur la pluralité de filaments et le stent, formant ainsi le stent-greffe (6),
le procédé de fabrication d'un stent-greffe (6) comprenant en outre la soumission du stent-greffe (6) à un processus de mise en forme et le retrait du stent-greffe façonné (6) de la bobine.

8. Procédé selon la revendication 7, dans lequel l'agencement de la pluralité de filaments et du stent (62) à l'extérieur du premier greffon (20) comprend :
disposer la pluralité de filaments à l'extérieur de la première greffe (20) ; et
gainer le stent (62) sur la pluralité de filaments.

9. Procédé selon la revendication 7, dans lequel l'agencement de la pluralité de filaments et du stent (62) à l'extérieur du premier greffon (20) comprend :
gainer le stent sur le premier greffon (20) ; et
entrelacer la pluralité de filaments à l'intérieur du stent (62).

10. Procédé selon la revendication 7, dans lequel l'agencement de la pluralité de filaments et du stent (62) à l'extérieur du premier greffon (20) comprend :
gainer le stent sur le premier greffon (20) ; et
disposer la pluralité de filaments à l'extérieur du stent (62).

11. Procédé selon la revendication 7, dans lequel, avant de soumettre l'endoprothèse (6) à un processus de mise en forme, le procédé comprend en outre :
envelopper l'endoprothèse (6) avec un film anti-poussière ; et
gainer un tube à sertir sur le film anti-poussière.

12. Procédé selon la revendication 6, dans lequel le premier greffon (20) et le deuxième greffon (23) sont formés par un procédé d'électrofilage.
